# EUROPÄISCHE PATENTSCHRIFT

(11) **EP 0 750 475 B1**
(45) Veröffentlichungstag und Bekanntmachung des Hinweises auf die Patenterteilung: **27.08.1997**
(21) Anmeldenummer: 95913853.8
(22) Anmeldetag: 22.03.1995
(51) Int. Cl.: A61B 17/34, A61M 31/00

(54) **VORRICHTUNG ZUR KONTROLLIERTEN PLAZIERUNG EINES TROKARS ODER EINER PUNKTIONSKANÜLE**
DEVICE FOR THE CONTROLLED POSITIONING OF A TROCAR OR A HOLLOW PUNCTURING NEEDLE
DISPOSITIF POUR LA MISE EN PLACE CONTROLEE D'UN TROCART OU D'UNE CANULE DE PONCTION

(30) Priorität: 19.04.1994 DE 4413520
(43) Veröffentlichungstag der Anmeldung: 02.01.1997
(73) Patentinhaber: WILLY RÜSCH AG, D-71394 Kernen-Rommelshausen (DE)
(72) Erfinder: AHMADZADEH, Massoud, D-48485 Neuenkirchen (DE)
(74) Vertreter: KOHLER SCHMID + PARTNER
(86) Internationale Anmeldenummer: DE9500386
(87) Internationale Veröffentlichungsnummer: WO9528132

(56) Entgegenhaltungen:
- US-A- 2 269 963
- US-A- 5 067 946
- US-A- 5 135 505
- US-A- 5 183 465
- US-A- 5 281 197

## Beschreibung

Die Erfindung betrifft eine Vorrichtung zur kontrollierten Plazierung eines Trokars oder einer Punktionskanüle.

Eine derartige Vorrichtung ist beispielsweise unter der Bezeichnung "ETHICON ENDOPATH" bekanntgeworden.

Bei der bekannten Vorrichtung ist der Trokarstössel von einem axial verschiebbaren Rohr umhüllt, das im Ausgangszustand auch die Trokarstösselspitze umhüllt. Das Rohr mit dem Trokarstössel ist in einem Schaft geführt und dort verrastbar gehalten. Soll der Schaft durch eine Gewebewandung hindurchgeführt werden, so wird das Rohr mit dem Trokarstössel in den Schaft gesteckt, und beide Teile werden an der patientenabgewandten Seite miteinander verrastet. Der Trokarstössel ragt in dieser Anordnung über das freie Ende des Schafts heraus, wobei die Trokarstösselspitze von dem axial verschiebbaren Rohr noch vollkommen abgedeckt und umhüllt ist.

Soll nun ein Durchbruch durch eine Gewebewandung oder dergleichen geschaffen werden, so wird das patientenseitige Ende des Schaftes mit dem daraus hervorstehenden Rohr auf die Gewebewandung aufgesetzt und durch die von Hand des Benutzers aufzubringende Kraftkomponente in axialer Richtung tritt die Trokarstösselspitze der Vorrichtung aus der Rohrumhüllung heraus und dringt in die anliegende Gewebewandung ein. Je nachdem, wie stark der Benutzer auf die bekannte Vorrichtung drückt, desto schneller dringt der Schaft mit dem Trokarstössel in die Gewebewandung ein.

Bei einer derartigen Arbeitsbewegung mit der bekannten Vorrichtung kann es zu unkontrollierten Auslenkungen des Trokarstösseis kommen, weil bei erhöhtem Widerstand auch mit einer erhöhten Kraft auf den Schaft gedrückt werden muß, damit dieser eine Gewebewandung durchdringen kann. Läßt nun der Widerstand sprunghaft nach, so kann möglicherweise nicht synchron dazu die aufgewandte Kraftkomponente für die Axialverschiebung reduziert werden, und der Schaft führt eine längere Axialbewegung als gewollt aus.

Aus der US-PS 5,067,946 ist eine verletzungsvermeidende Nadelvorrichtung bekannt, mit der eine Nadel über ein Verstellrad aus einem Gehäuse herausgeschoben werden kann. Die Nadel wird in Verbindung mit Bluttransfusionen und bei intravenösen Anwendungen eingesetzt. Zähne des Verstellrades greifen in Zähne einer axial in der Vorrichtung verschiebbaren Halterung, die die Nadel hält. Wird am Verstellrad gedreht, so verschiebt sich die Nadel.

Durch die US-PS 2,269,963 ist eine Implantationsvorrichtung bekanntgeworden, mit der radioaktive Kapseln kontrolliert aus einem Magazin in einem Hohlraum ausgesetzt werden können. Das mit den Kapseln gefüllte Rohr muß von Hand und möglicherweise auch gegen eventuell auftretende Widerstände in den Hohlraum eingeschoben werden. Ist das freie Ende des Rohres bestimmungsgemäß plaziert, so kann eine oder mehrere Kapseln kontrolliert ausgesetzt werden.

In der US-PS 5,183,465 ist eine Vorrichtung beschrieben, über die eine Nadel oder ein Trokar durch die Bauchdecke hindurchgeführt werden können. Die Nadel oder der Trokar sind in einem Haltegestell geführt und verschieblich gehalten. Das Haltegestell mit der Nadel wird auf die Bauchdecke von aussen aufgesetzt und am Haltegestell ist eine Schraube drehbar befestigt, deren freies Ende mit der Nadel beziehungsweise dem Trokar zusammenwirkt. Wird an der Schraube gedreht, so verschiebt sich bei einer Drehrichtung die Schraube in die Halterung hinein und drückt dabei die Nadel beziehungsweise den Trokar aus der Halterung heraus. Muß die Nadelspitze oder der Trokar einen mehr oder weniger starken Widerstand überwinden, so muß ebenfalls unterschiedlich fest an der Verstellschraube gedreht werden.

Die Aufgabe der vorliegenden Erfindung ist es deshalb, eine Vorrichtung zur kontrollierten Plazierung eines Trokars oder einer Punktionskanüle zu entwickeln, die von den Benutzern der Vorrichtung keine unterschiedlich starke Kraftanwendung verlangt, sondern eine gleichmäßige, kontrollierte Axialbewegung des Schaftes mit dem Trokarstössel unabhängig von der dem Trokarstössel entgegenwirkenden Widerstandskraft ermöglicht.

Die Aufgabe wird erfindungsgemäß dadurch gelöst, daß der Trokar oder die Punktionskanüle in der Vorrichtung axial verfahrbar gehalten ist und über Auslösemittel, die einen ortsfesten Haltegriff und einen beweglichen Haltegriff aufweisen, wobei über den Auslenkweg des beweglichen Haltegriffs zum ortsfesten Haltegriff die Länge des Verfahrweges des Trokars oder der Punktionskanüle in eine erste zum Patienten gerichtete Richtung definiert ist, in Bezug auf eine Anlagefläche der Vorrichtung in die erste Richtung schrittweise bewegbar ist.

Die erfindungsgemäße Vorrichtung hat damit den wesentlichen Vorteil, daß beliebige Trokarsysteme unabhängig von ihrer Größe, das heißt, unabhängig von ihrer Größe des Schaftdurchmessers, immer mit konstanter Kraftaufwendung vom Benutzer plaziert werden können. Über die Anlagefläche kann die erfindungsgemäße Vorrichtung sicher an der gewünschten Stelle angelegt werden und über die Auslösemittel wird der Trokar oder die Punktionskanüle bewegt. Je nachdem mit welchen Mitteln die Vorrichtung ausgelenkt wird und welche kraftübertragenden Vorrichtungsteile vorgesehen sind, kann eine mehr oder weniger starke Widerstandskraft, die sich beim Verfahren des Trokars oder der Punktionskanüle ergibt, überwunden werden. In der Regel ist die Anlagefläche sehr viel größer als der zu plazierende Trokar, sodaß ein sicheres Auflager geschaffen ist, das ein Eindringen des Trokars stets an der gewünschten Stelle ermöglicht. Ein ungewolltes Verrutschen der Vorrichtung beim Einstich des Trokars in die Gewebewandung wird damit sicher verhindert. Die Auslösemittel können mechanisch oder motorisch bewegt sein, sodaß die erfindungsgemäße Vorrichtung für eine breite Anwendung bei stets konstantem Kraftaufwand durch den Benutzer geeignet ist.

Die Auslösemittel weisen einen ortsfesten Haltegriff und einen beweglichen Haltegriff auf, wobei über den Auslenkweg des beweglichen Haltegriffs zum ortsfesten Haltegriff die Länge des Verfahrweges des Trokars oder der Punktionskanüle in eine erste zum Patienten gerichtete Richtung definiert ist.

Dies hat den Vorteil, daß über die Betätigung der Haltegriffe ein exakter Verfahrweg des Trokars oder der Punktionskanüle vorgebbar ist. Die Haltegriffe sind derart ausgelegt, daß sie einer Handbewegung des Benutzers angepaßt sind und ohne erhöhte Kraftaufwendung ausgelenkt werden können. Dabei kann ein großer Auslenkweg der Haltegriffe einem kurzen Verfahrweg des Trokars oder der Funktionskanüle entsprechen. Die Übersetzung vom Verschwenkungsweg der Haltegriffe zum Verfahrweg des Trokars oder der Funktionskanüle ist frei vorgebbar.

In weiterer Ausgestaltung der Erfindung ist der zu plazierende Trokar oder die Punktionskanüle in eine Öffnung der Vorrichtung einschiebbar und durch die Vorrichtung hindurch verschiebbar. Weiterhin ist über die Auslenkung des beweglichen Haltegriffs in Richtung des ortsfesten Haltegriffs der Trokar oder die Punktionskanüle mit Abschnitten eines Durchbruchs einer über den beweglichen Haltegriff auslenkbaren Platte verkantbar und über die Auslenkung der Platte ist der Trokar oder die Punktionskanüle in die erste Richtung bewegbar.

Dies hat den Vorteil, daß handelsübliche Trokare beziehungsweise Trokarsysteme beziehungsweise Punktionskanülen in Verbindung mit der erfindungsgemäßen Vorrichtung verwendet werden können. Der Trokar wird in die erfindungsgemäße Vorrichtung eingeführt und über die erfindungsgemäße Vorrichtung in axialer Richtung bewegt. Besonders einfach und effektiv wird die Bewegung des Trokars in axialer Richtung dadurch erreicht, wenn eine Platte bei ihrer Auslenkbewegung den Trokar umgreift und aufgrund von bestehenden Reibkräften zwischen dem Trokar und der Platte den Trokar axial auslenkbar bewegen kann. Die Platte wird in der erfindungsgemäßen Vorrichtung so verschwenkt, daß die Kanten eines Durchbruchs in der Platte mit der Außenumfangsfläche des Trokars verkanten und bei einem weiteren Verschwenken der Platte verfährt der Trokar in die erste Richtung.

In weiterer Ausgestaltung der Erfindung ist die Platte in eine zweite der ersten Richtung entgegengesetzte Richtung belastet und die maximale Auslenkung der Platte ist durch den Auslenkweg vom beweglichen Haltegriff zum ortsfesten Haltegriff begrenzt.

Dies hat den Vorteil, daß die Platte durch die Feder immer selbsttätig in eine Lage gedrückt werden kann, in der ein Trokar oder eine Punktionskanüle verkantungsfrei in die erfindungsgemäße Vorrichtung und durch den Durchbruch in der Platte hindurch geführt werden kann. Ebenfalls wird über die Feder die Platte in ihre Ausgangsstellung (Ruhelage) zurückgedrückt. Nur über die Haltegriffe kann die Platte entgegen der Federkraft ausgelenkt werden. Damit ist ein Druckpunkt geschaffen, den der Benutzer überwinden muß, wenn er den Trokar oder die Punktionskanüle axial auslenken möchte.

In weiterer bevorzugter Ausgestaltung der Erfindung ist die Vorrichtung auf der Oberseite zur Freilegung der Öffnung aufklappbar.

Dies hat den Vorteil, daß bei plaziertem Trokar oder plazierter Punktionskanüle die Vorrichtung auch dann problemfrei wieder von dem plazierten Trokar beziehungsweise von der plazierten Punktionskanüle entfernt werden kann, wenn das patientenabgewandte Ende des Trokars beziehungsweise der Punktionskanüle größer ist als die Öffnung an der erfindungsgemäßen Vorrichtung, durch die der Trokar beziehungsweise die Punktionskanüle hindurchgeführt wird.

In weiterer Ausgestaltung der Erfindung weist die Vorrichtung den Haltegriffen gegenüberliegend eine am Gehäuse der Vorrichtung ausgebildete Halterung auf.

Dies hat den Vorteil, daß bei einem Plazierungsvorgang der Trokar oder die Punktionskanüle sicher geführt gehalten werden kann und die erfindungsgemäße Vorrichtung kann nicht aus ihrer gewünschten Lage verrutschen.

In weiterer Ausgestaltung der Erfindung weist der Trokar oder die Punktionskanüle auf einem begrenzten Außenabschnitt längs der axialen Erstreckung Einkerbungen auf, die in der Vorrichtung in Verrastelemente eingreifen, wobei das erste Verrastelement am beweglichen Haltegriff befestigt und mit dem beweglichen Haltegriff verschwenkbar ist und das zweite Verrastelement ist am ortsfesten Gehäuse der Vorrichtung angeordnet.

Diese Ausgestaltung hat den Vorteil, daß bei einem weiteren Ausführungsbeispiel der erfindungsgemäßen Vorrichtung das axiale Verfahren eines Trokars dann sicher erfolgen kann, wenn dieser Einkerbungen aufweist, die mit den Verrastelementen der Vorrichtung zusammenwirken können. Die Verrastelemente haben eine Auflaufschräge und sind derart federbelastet, daß sie immer in eine Ausgangsstellung selbsttätig zurückkehren möchten, von der die Axialbewegung des Trokars oder der Punktionskanüle ausgeht. Das zweite Verrastelement hat die Aufgabe, den Trokar oder die Punktionskanüle in der jeweiligen Stellung zu halten und verhindert eine unkontrollierte Bewegung des Trokars oder der Punktionskanüle entgegen der ersten Richtung.

In einer weiteren Ausgestaltung der Erfindung ist der Trokar oder die Punktionskanüle mit dem patientenabgewandten Ende in einem axial verfahrbaren Schlitten der Vorrichtung ortsfest haltbar gelagert, der Schlitten ist in beliebig langen vorgebbaren Wegabschnitten innerhalb der Vorrichtung verschiebbar, der Trokar oder die Punktionskanüle tritt mit dem patientenseitigen Ende beim Verfahren des Schlittens in der ersten Richtung aus der Vorrichtung heraus, an der Vorrichtung ist einenends die Anlagefläche ausgebildet, aus der der Trokar oder die Punktionskanüle beim Verfahren in der ersten Richtung heraustritt und anderenends ist ein ortsfester und ein beweglicher Haltegriff ausgebildet, wobei über den beweglichen Haltegriff die Verschiebung des Schlittens in die erste Richtung auslösbar ist.

Dies hat den Vorteil, daß nur der Schlitten und die Anlagefläche an den zu plazierenden Trokar oder die Punktionskanüle angepaßt werden müssen. Bei dieser Ausführungsform der erfindungsgemäßen Vorrichtung läßt sich die Anlagefläche breitflächig ausbilden, sodaß eine sichere Auflage an der Gewebewandung gegeben ist. Die Vorrichtung ist so ausgebildet, daß der Trokar oder die Punktionskanüle in der Ausgangsstellung komplett innerhalb der Vorrichtung gelagert ist, sodaß bei der Handhabung der Vorrichtung auch dann keine Verletzungsgefahr besteht, wenn die Trokarstösselspitze beziehungsweise die Kanülenspitze nicht zusätzlich abgedeckt ist. Werden die Auslösemittel bewegt, so tritt der Trokar oder die Punktionskanüle aus der Vorrichtung in vorgegebenen Schrittweiten aus und dringt in die unmittelbar angrenzende Gewebewandung ein. Nach Beendigung der Plazierung wird der Schlitten geöffnet und die Vorrichtung wird vom Trokar beziehungsweise der Punktionskanüle getrennt.

Am patientenabgewandten Ende der Vorrichtung ist ein ortsfester und ein beweglicher Haltegriff ausgebildet, wobei über den beweglichen Haltegriff die schrittweise oder kontinuierliche Verschiebung des Schlittens in die erste Richtung auslösbar ist. Damit läßt sich der Trokar beziehungsweise die Punktionskanüle in kleinsten Schritten axial exakt verfahren.

In weiterer Ausgestaltung der Erfindung ist der Schlitten in einem halbschalenförmigen Gehäuse der Vorrichtung geführt gelagert, und der Schlitten weist eine Einspannvorrichtung für das patientenabgewandte Ende des Trokars oder der Punktionskanüle auf.

Dies hat den Vorteil, daß der Schlitten effektiv mit einfachen und leicht zugänglichen Mitteln sicher axial bewegbar ist und gerichtet geführt werden kann. Über die Einspannvorrichtung lassen sich beliebig ausgeformte Enden von einem Trokar oder einer Punktionskanüle sicher halten, schnell lageausgerichtet einspannen und ebenfalls aus der Einspannvorrichtung entfernen.

In Weiterbildung der erfindungsgemäßen Vorrichtung ist das halbschalenförmige Gehäuse am patientenabgewandten Ende um die Längsachse der Vorrichtung in Pfeilrichtungen drehbar gelagert.

Dies hat den Vorteil, daß die Trokarstösselspitze beziehungsweise die Punktionskanülenspitze unabhängig von der Lage der Vorrichtung ausgerichtet werden kann. Somit können die Auslösemittel immer bestmöglich zu dem Benutzer gestellt sein.

In weiterer Ausgestaltung der erfindungsgemäßen Vorrichtung ist über den Auslenkweg des beweglichen Haltegriffs zum ortsfesten Haltegriff eine Führungsstange verschiebbar, die mit dem Schlitten eine feste Verbindung aufweist. Die Führungsstange ermöglicht die exakte Führung des Schlittens und die Führungsstange kann unabhängig von der Ausgestaltung des zu plazierenden Trokars oder der zu plazierenden Punktionskanüle zur Kraftübertragung von den Auslösemitteln zu dem Schlitten ausgebildet werden. Die axiale Vorschubbewegung des Trokars wird nicht unmittelbar auf den Trokar oder die Punktionskanüle übertragen, sondern auf den Schlitten, der den Trokar oder die Punktionskanüle hält.

In weiterer Ausgestaltung der Erfindung ist die Führungsstange in jeder Positionslage unverrückbar gehalten, und sie ist ausschließlich über die Auslösemittel in die erste Richtung bewegbar.

Dies hat den Vorteil, daß sich während der Plazierung des Trokars beziehungsweise der Punktionskanüle der Trokar beziehungsweise die Punktionskanüle nicht ungewollt entgegen der ersten Richtung bewegen kann. Auch wenn der Plazierungsvorgang unterbrochen werden sollte, bleibt der Trokar beziehungsweise die Punktionskanüle an der Stelle stehen, in die sie verfahren worden sind.

Weist die Anlagefläche einen Führungsabschnitt beispielsweise eine Ausnehmung auf, in dem der Trokar beziehungsweise die Punktionskanüle geführt ist, so ist eine besonders sichere Führung des Trokars beziehungsweise der Punktionskanüle gegeben. An zwei Lagerpunkten liegt der Trokar während des gesamten Plazierungsvorgangs auf.

In weiterer Ausgestaltung der erfindungsgemäßen Vorrichtung weisen die Haltegriffe einen Entriegelungsmechanismus, beispielsweise einen Hebel auf, der im aktivierten Zustand eine Verschiebung des Schlittens in eine zweite Richtung ermöglicht.

Dies hat den Vorteil, daß die Führungsstange mit dem Schlitten immer dann zurückgeführt werden kann, wenn der Entriegelungsmechanismus betätigt wird. Wird der Hebel aus einer verkanteten Stellung zur Führungsstange gelöst, kann die Führungsstange entgegen der Plazierungsrichtung bewegt werden. Der Hebel ist derart federbelastet, daß er stets eine Bewegung der Führungsstange in Plazierungsrichtung zuläßt, aber eine Bewegungsrichtung in die zweite Richtung verhindert. In die zweite Richtung kann die Führungsstange nur dann bewegt werden, wenn der Hebel entgegen seiner Federbelastung entriegelt wird.

In bevorzugter Ausgestaltung der Erfindung weist der Schlitten Ausnehmungen auf, in die das patientenabgewandte Ende des Trokars oder der Punktionskanüle weitgehend formschlüßig einlegbar sind, und der Trokar oder die Punktionskanüle ist über einen Schnellverschluß in den Ausnehmungen richtungsstabil geführt gehalten. Im Schlitten können unterschiedlich auswechselbare Einsätze vorgesehen sein, die jeweils an die patientenabgewandten Enden eines Trokars beziehungsweise einer Punktionskanüle angepaßt sind. Damit kann die erfindungsgemäße Vorrichtung weitgehend in Verbindung mit handelsüblichen Trokarsystemen beziehungsweise Punktions- kanülen eingesetzt werden.

Es ist ebenfalls denkbar, daß der Schlitten und/oder die Anlagefläche in der Vorrichtung austauschbar gehalten sind. Ist auch die Anlagefläche austauschbar, so kann jeweils bei Bedarf eine Anlagefläche eingesetzt werden, die eine Ausnehmung aufweist, die an den jeweiligen Trokar, beziehungsweise an die zu plazierende Punktionskanüle angepaßt ist.

Ist zwischen dem Schlitten und den Auslösemitteln ein Abstandshalter vorgesehen, so kann die eine Endstellung eines Schlittens immer leicht angefahren werden. Der Schlitten wird in entriegelter Stellung bis zum Anschlag an den Abstandshalter zurückgefahren. Der Schlitten befindet sich dann in einer Stellung, in der er einen Trokar beziehungsweise eine Punktionskanüle vollkommen aufnehmen kann, das heißt, das patientenzugewandte Ende des Trokars beziehungsweise der Punktionskanüle steht nicht über die Vorrichtung vor.

Werden die beschriebenen Vorrichtungen aus dampfsterilisierbaren Werkstoffen hergestellt und sind die einzelnen Komponenten der Vorrichtung einsehbar und zugänglich angeordnet, so können sie schnell und effektiv gereinigt werden. Die Vorrichtung kann somit mehrfach eingesetzt werden.

Weitere Vorteile ergeben sich aus der Beschreibung und der beigefügten Zeichnung. Ebenso können die vorstehend genannten und die noch weiter aufgeführten Merkmale erfindungsgemäß jeweils einzeln oder in beliebigen Kombinationen miteinander verwendet werden. Die erwähnten Ausführungsformen sind nicht als abschließende Aufzählung zu verstehen, sondern haben vielmehr beispielhaften Charakter.

Die Erfindung ist in der Zeichnung dargestellt und wird anhand von Ausführungsbeispielen in der Zeichnung näher erläutert. Es zeigt:
- Fig. 1: eine erfindungsgemäße Vorrichtung zur Plazierung einer Punktionskanüle, wobei eine handelsübliche Punktionskanüle in die Vorrichtung eingeschoben wird;
- Fig. 2: eine Punktionskanüle, wie sie in der Fig. 1 verwendet wird;
- Fig. 3: eine Funktionsdarstellung der Vorrichtung nach Fig. 1, wobei die erfindungsgemäße Vorrichtung teilweise aufgeschnitten gezeigt ist;
- Fig. 4: ein weiteres Funktionsprinzip einer erfindungsgemäßen Vorrichtung für die Plazierung von einem Trokar beziehungsweise einer Punktionskanüle mit Einkerbungen;
- Fig. 5: eine Punktionskanüle, wie sie beispielsweise in einer Vorrichtung gemäß Fig. 4 verwendet wird;
- Fig. 6: eine weitere Ausführungsform einer erfindungsgemäßen Vorrichtung zur Plazierung von einem Trokar beziehungsweise einer Punktionskanüle in räumlich gezeigter Darstellung.

Die Figuren zeigen den erfindungsgemäßen Gegenstand stark schematisiert und sind nicht maßstäblich zu verstehen.

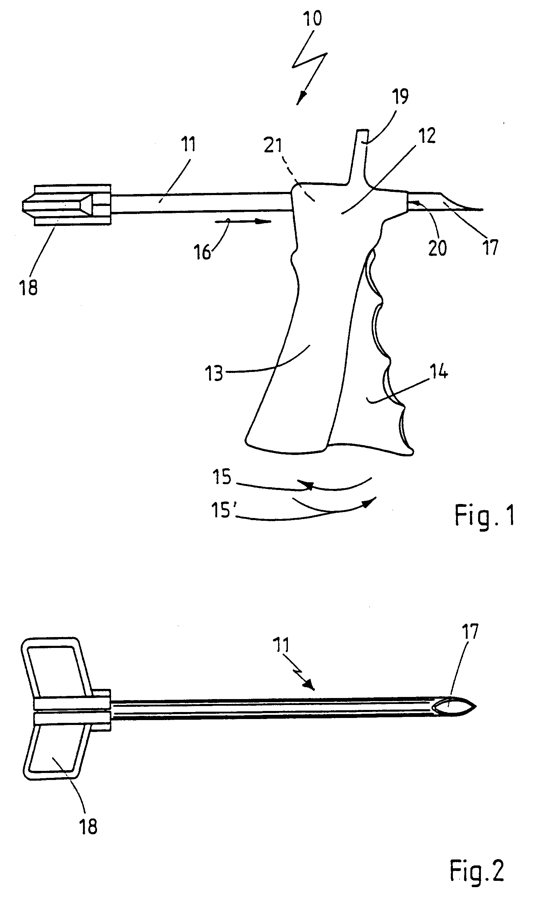

Fig. 1 zeigt eine Vorrichtung 10 zur kontrollierten Plazierung einer Punktionskanüle 11. Die Vorrichtung 10 weist ein Gehäuse 12 auf, an dem ein ortsfester Haltegriff 13 ausgeformt ist. Im ortsfesten Haltegriff 13 ist ein beweglicher Haltegriff 14 gehalten. Der bewegliche Haltegriff 14 kann in Pfeilrichtung 15 auf den ortsfesten Haltegriff 13 unter Überwindung eines Federdrucks hinbewegt werden. Der bewegliche Haltegriff 14 bewegt sich druckentlastet selbsttätig in Pfeilrichtung 15'.

Die zu plazierende Punktionskanüle 11 kann in das Gehäuse 12 in erster Richtung 16 eingeschoben werden. Die Punktionskanüle 11 selbst weist eine Spitze 17 auf, die zum Patienten hin ausgerichtet ist und am patientenabgewandten Ende ist die Punktionskanüle 11 als flügelförmiges Ende 18 ausgebildet.

An dem Gehäuse 12 ist weiterhin eine Halterung 19 ausgeformt und auf der patientenzugewandten Seite ist an der Vorrichtung 10 eine Anlagefläche 20 ausgebildet. Die Vorrichtung 10 weist eine Öffnung 21 auf, in die die Punktionskanüle 11 eingeschoben werden kann. Wird die Punktionskanüle 11 in die Öffnung 21 eingeschoben, so durchdringt sie einen Durchbruch 22 einer Platte 23, die mit dem beweglichen Haltegriff 14 zusammenwirkt. Wird der bewegliche Haltegriff 14 in Richtung 15 auf den ortsfesten Haltegriff 13 bewegt, so verschiebt sich die Punktionskanüle 11 in der ersten Richtung 16.

Fig. 2 zeigt eine Gesamtansicht einer in Fig. 1 verwendeten Punktionskanüle 11. Die handelsübliche Punktionskanüle 11 ist als Hohlnadel ausgebildet und weist eine Spitze 17 an der patientenzugewandten Seite auf. Am dazu diametralen Ende ist die Punktionskanüle 11 als flügelförmiges Ende 18 ausgebildet. Die Flügel können unter geringer Kraftaufwendung aufgeklappt werden. Dabei wird die Punktionskanüle 11 in bekannter Weise axial gespalten.

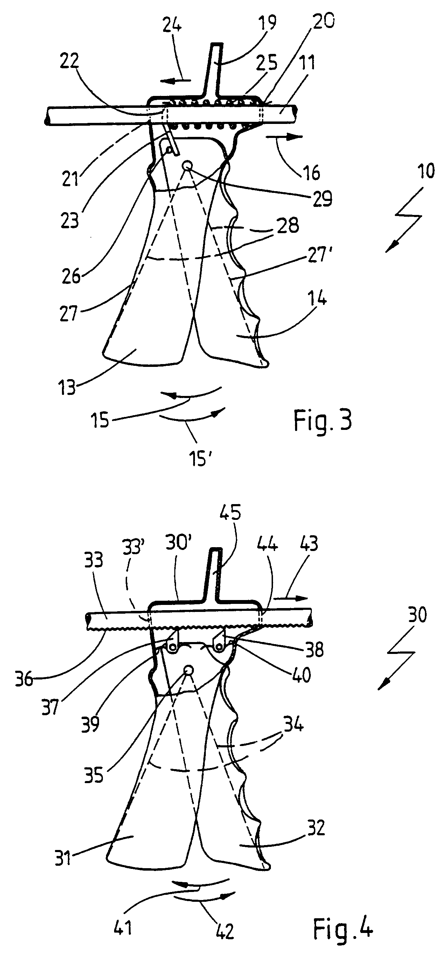

Fig. 3 zeigt das Funktionsprinzip der Vorrichtung 10, wie sie in Fig. 1 gezeigt ist.

Von der Punktionskanüle 11 ist nur ein Abschnitt gezeigt, der die Vorrichtung 10 durchdringt.

Soll die Vorrichtung 10 eingesetzt werden, so ist die Punktionskanüle 11 soweit zurückgezogen, daß sie nicht über die Anlagefläche 20 vorsteht. Die Anlagefläche 20 wird auf einer Gegenfläche aufgelegt. Danach hält der Benutzer die Vorrichtung 10 mit einer Hand an den Haltegriffen 13, 14 und mit der anderen Hand an der Halterung 19. Werden die Haltegriffe 13, 14 zusammengedrückt, so bewegt sich die Punktionskanüle 11 in die erste Richtung 16. In der in der Fig. 3 gezeigten Haltegriffstellung kann die Punktionskanüle 11 in zweiter Richtung 24 zurückgezogen werden. Die Punktionskanüle 11 ist in der Vorrichtung 10 von dem Durchbruch 22 der Platte 23 umgriffen. Die Platte 23 ist über eine Feder 25 druckbelastet. Die Federbelastung wirkt der ersten Richtung 16 entgegen. Die Platte 23 ist beweglich mit einem Stift 26 des beweglichen Haltegriffes 14 verbunden. Werden die Haltegriffe 13, 14 zusammengedrückt, so verkantet der Durchbruch 22 an seiner Außenkontur mit der Außenumfangsfläche der Punktionskanüle 11 und führt die Punktionskanüle 11 unter Reibschluß in die erste Richtung 16. Werden die Haltegriffe 13, 14 kraftentlastet, so wird der bewegliche Haltegriff 13 mittels der Schenkel 27, 27' einer Feder 28, die in einer Lagerung 29 gehalten ist, in die in der Fig. 3 gezeigten Stellung gedrückt. Dabei wird die Platte 23 zurückverfahren, die Platte 23 wird über die Feder 25 in eine Ausgangsstellung gedrückt. Werden nun erneut die Haltegriffe 13, 14 zusammengedrückt, so wird die Punktionskanüle 11 erneut über die Platte 23 in erster Richtung 16 verschoben.

Fig. 4 zeigt eine weitere Ausführungsform einer Vorrichtung 30, die teilweise aufgeschnitten gezeigt ist. Die Vorrichtung 30 ist als Gehäuse 30' mit einem ortsfesten Haltegriff 31 und einem beweglichen Haltegriff 32 ausgebildet. Mit der Vorrichtung 30 kann eine Punktionskanüle 33 kontrolliert plaziert werden. Die Punktionskanüle 93 ist in eine Öffnung 33' des Gehäuses 30' einführbar. Die Haltegriffe 31, 32 werden über eine Feder 34, die in einer Lagerung 35 gehalten ist, auseinandergedrückt.

Mit der Vorrichtung 30 können Punktionskanülen 33 beziehungsweise Trokarsysteme plaziert werden, die Einkerbungen 36 aufweisen. Die Einkerbungen 36 müssen zumindest über einen Teilabschnitt am Außenumfang der Punktionskanüle 33 ausgebildet sein. Die Einkerbungen 36 greifen in Verrastelemente 37, 38 ein, die über Federmittel 39, 40 belastet sind. Das Verrastelement 37 ist mit dem beweglichen Haltegriff 32 verbunden und das Verrastelement 38 ist im Gehäuse 30' gelagert gehalten. Der bewegliche Haltegriff 32 kann in Pfeilrichtung 41 beziehungsweise 42 bewegt werden. Wird der bewegliche Haltegriff 32 in Pfeilrichtung 41 bewegt, so bewegt sich die Punktionskanüle 33 in eine erste Richtung 43.

Soll mittels der Vorrichtung 30 eine Punktionskanüle 33 plaziert werden, so wird die Vorrichtung 30 über eine Anlagefläche 44 an der gewünschten Gegenfläche aufgelegt. Ein Benutzer hält die Vorrichtung 30 an den Haltegriffen 31, 32 und an einer Halterung 45. Werden die Haltegriffe 31, 32 zusammengedrückt, so wird die Punktionskanüle 33 über das Verrastelement 37 in Pfeilrichtung 43 geführt. Das stationär gelagerte Verrastelement 38 bewegt sich ebenfalls in Pfeilrichtung 43. Wird der bewegliche Haltegriff 32 in Pfeilrichtung 42 verschwenkt, so verfährt das Verrastelement 37 über eine am Verrastelement 37 ausgebildete Schrägfläche in seine Ausgangsstellung zurück und verrastet dort in einer Einkerbung 36 der Punktionskanüle 33. Werden nun erneut die Haltegriffe 31, 32 zusammengedrückt, so kann die Punktionskanüle 33 erneut in erster Richtung 43 verfahren werden.

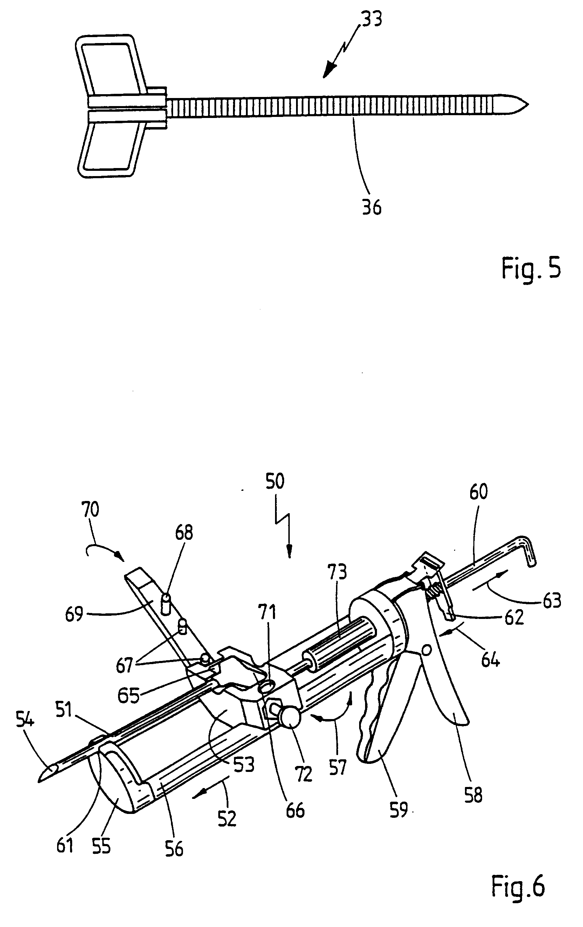

Fig. 5 zeigt die Punktionskanüle 33 von der Unterseite, wie sie in der Vorrichtung 30 verwendet wird. Auf der Unterseite sind an der Punktionskanüle 33 Einkerbungen 36 ausgebildet, die in der Vorrichtung 30 in Verrastelemente 37, 38 eingreifen können. Über die Flügel am patientenabgewandten Ende der Punktionskanüle 33 läßt sich die Punktionskanüle 33 bei Bedarf in axialer Richtung spalten.

Die in Fig. 3 und 4 gezeigten Vorrichtungen lassen sich bei Bedarf, dies ist in den Figuren 3 und 4 nicht gezeigt, im Bereich der Oberseite öffnen, damit nach einer Plazierung der Punktionskanüle die Vorrichtung von der plazierten Punktionskanüle getrennt werden kann.

Fig. 6 zeigt ein weiteres Ausführungsbeispiel einer erfindungsgemäßen Vorrichtung 50 zur kontrollierten Plazierung einer Punktionskanüle 51 oder eines Trokars. Über die Vorrichtung 50 läßt sich die Punktionskanüle 51 in eine erste Richtung 52 kontrolliert verfahren. Die Punktionskanüle 51 ist mit dem vom Patienten abgewandten Ende in einem verfahrbaren Schlitten 50 gehalten. Die Punktionskanüle 51 liegt über eine Spitze 54 an einer Anlagefläche 55. Die Anlagefläche 55 ist in einem halbschalenförmigen Gehäuse 56 der Vorrichtung 50 auswechselbar gehalten. Das halbschalenförmige Gehäuse 56 ist in Pfeilrichtungen 57 drehbar. An der Vorrichtung 50 ist ein erster ortsfester Haltegriff 58 ausgebildet in dem ein zweiter beweglicher Haltegriff 59 gelagert ist. Der bewegliche Haltegriff 59 kann bei Bewegung eine Führungsstange 60 in Pfeilrichtung 52 verschieben. In der Anlagefläche 55 ist eine Ausnehmung 61 ausgebildet, die eine Führung für die Punktionskanüle 51 darstellt.

Die Führungsstange 60 durchdringt in der Vorrichtung 50 einen Hebel 62, der die Führungsstange 60 immer in der augenblicklichen Lage hält. Die Führungsstange 60 kann dann in eine zweite Richtung 63 von Hand verfahren werden, wenn der Hebel 62 in Pfeilrichtung 64 entgegen dem Druck einer Feder verschwenkt wird (Entriegelung).

Die Führungsstange 60 ist mit dem Schlitten 53 verbunden, der geführt im halbschalenförmigen Gehäuse 56 innerhalb des Gehäuses 56 verfahren kann. Wird die Führungsstange 60 verfahren, so wird auch der Schlitten 53 verfahren. Im Schlitten 53 sind Ausnehmungen 65 für ein flügelartiges Ende 66 der Punktionskanüle 51 ausgebildet. Die Ausnehmungen 65 können beliebige Kontouren aufweisen, das heißt, die Ausnehmungen 65 sind auch auswechselbar zu gestalten, sodaß beliebige Enden von Trokarsystemen in die Ausnehmungen 65 möglichst formschlüßig einlegbar sind. Das vom Patienten abgewandte Ende einer Punktionskanüle beziehungsweise eines Trokars wird in den Ausnehmungen 65 über Haltestife 67 gehalten, sobald ein Verriegelungsstift 68 einer Platte 69 in Pfeilrichtung 70 verschwenkt in einer Aufnahme 71 von einem verschiebbaren Stift 72 fixiert ist. Über den verschiebbaren Stift 72 kann die Platte 69 bei Bedarf entriegelt werden, und die Platte 69 kann entgegen der Pfeilrichtung 70 wieder verschwenkt werden. Die plazierte Punktionskanüle beziehungsweise der plazierte Trokar kann dann aus den Ausnehmungen 65 herausgenommen werden.

Innerhalb des halbschalenförmigen Gehäuses 56 ist an der Führungsstange 60 ein Abstandshalter 73 vorgesehen, der eine Endposition des Schlittens 53 definiert. Der Abstandshalter 73 ist an die Länge einer zu plazierenden Punktionskanüle beziehungsweise eines Trokars angepaßt. Die Vorrichtung nach Fig. 6 weist innerhalb der Haltegriffe 58, 59 eine verschwenkbare Platte auf, wie sie beispielsweise in Fig. 3 gezeigt und beschrieben ist. Der Hebel 62 wird über eine Feder in eine Position gedrückt, die verhindert, daß die Führungsstange 60 bei federbelastetem Hebel in Pfeilrichtung 63 bewegt werden kann. Wird der Hebel 62 entgegen der Federbelastung ausgelenkt, so kann die Führungsstange 60 in Pfeilrichtung 63 verfahren werden.

Eine Vorrichtung 10 hält eine Punktionskanüle 11, die innerhalb einer Öffnung 21 in der Vorrichtung 10 in eine erste Richtung 16 kontrolliert verfahren werden kann. Der bewegliche Haltegriff 14 ist unter Druck in Pfeilrichtung 15 bewegbar. Wird der bewegliche Haltegriff 14 in Pfeilrichtung 15 bewegt, so verschiebt sich die Punktionskanüle 11 in die erste Richtung 16. Die Vorrichtung 10 ist über eine Anlagefläche 20 an einer Gegenfläche zu plazieren. Die Punktionskanüle 11 ist in der Vorrichtung 10 axial verfahrbar gehalten und über den beweglichen Haltegriff 14 in eine erste Richtung 16 kontrolliert bewegbar.

## Patentansprüche

1. Vorrichtung zur kontrollierten Plazierung eines Trokars oder einer Punktionskanüle (11; 33; 51), wobei der Trokar oder die Punktionskanüle (11; 33; 51) in der Vorrichtung (10; 30; 50) axial verfahrbar gehalten ist und über Auslösemittel, die einen ortsfesten Haltegriff (13; 31; 58) und eine beweglichen Haltegriff (14; 32; 59) aufweisen, wobei über den Auslenkweg des beweglichen Haltegriffs (14; 32; 59) zum ortsfesten Haltegriff (13; 31; 58) die Länge des Verfahrweges des Trokars oder der Punktionskanüle (11; 33; 51) in eine erste zum Patienten gerichtete Richtung (16; 43; 52) definiert ist, in Bezug auf eine Anlagefläche (20; 44; 55) der Vorrichtung (10; 30; 50) in die erste Richtung (16; 43; 52) schrittweise bewegbar ist.

2. Vorrichtung zur kontrollierten Plazierung eines Trokars oder einer Punktionskanüle (11) nach Anspruch 1, dadurch gekennzeichnet, daß der zu plazierende Trokar oder Punktionskanüle (11) in eine Öffnung (21) der Vorrichtung (10) einschiebbar und durch die Vorrichtung (10) hindurch verschiebbar ist, daß über die Auslenkung des beweglichen Haltegriffs (14) in Richtung des ortsfesten Haltegriffs (13) der Trokar oder die Punktionskanüle (11) mit Abschnitten eines Durchbruchs (22) einer über den beweglichen Haltegriff (14) auslenkbaren Platte (23) verkantbar ist und daß über die Auslenkung der Platte (23) der Trokar oder die Punktionskanüle (11) in die erste Richtung (16) bewegbar ist.

3. Vorrichtung zur kontrollierten Plazierung eines Trokars oder einer Punktionskanüle (11) nach einem der Anspruch 1 oder 2, dadurch gekennzeichnet, daß die Platte (23) in eine zweite der ersten Richtung (16) entgegengesetzte Richtung (24) federbelastet ist und die maximale Auslenkung der Platte (23) durch den Auslenkweg vom beweglichen Haltegriff (14) zum ortsfesten Haltegriff (13) begrenzt ist.

4. Vorrichtung zur kontrollierten Plazierung eines Trokars oder einer Punktionskanüle (11) nach einem der Ansprüche 2 oder 3, dadurch gekennzeichnet, daß die Vorrichtung (10) auf der Oberseite zur Freilegung der Öffnung (21) aufklaßßbar ist.

5. Vorrichtung zur kontrollierten Plazierung eines Trokars oder einer Punktionskanüle (11; 33) nach einem der Ansprüche 2 bis 4, dadurch gekennzeichnet, daß die Vorrichtung (10; 30) den Haltegriffen (13; 14; 31; 32) gegenüberliegend eine am Gehäuse (12; 30') der Vorrichtung (10; 30) ausgebildete Halterung (19; 45) aufweist.

6. Vorrichtung zur kontrollierten Plazierung eines Trokars oder einer Punktionskanüle (33) nach Anspruch 1, dadurch gekennzeichnet, daß der Trokar oder die Punktionskanüle (33) auf einem begrenzten Außenumfangsabschnitt längs der axialen Erstreckung Einkerbungen (36) aufweist, die in der Vorrichtung (30) in Verrastelemente (37, 38) angreifen, wobei das erste Verrastelement (37) am beweglichen Haltegriff (32) befestigt und mit dem beweglichen Haltegriff (32) verschwenkbar ist und das zweite Verrastmittel (38) am ortsfesten Gehäuse (30') der Vorrichtung (30) angeordnet ist.

7. Vorrichtung zur kontrollierten Plazierung eines Trokars oder einer Punktionskanüle nach einem der Ansprüche 1 bis 6, dadurch gekennzeichnet, daß die Auslösemittel (13, 14; 31, 32) über eine Feder (28; 34) auseinander gehalten sind und daß die Auslösemittel (13, 14; 31, 32) entgegen der Federkraft der Federn (28; 34) aufeinander zu bewegbar sind.

8. Vorrichtung zur kontrollierten Plazierung eines Trokars oder einer Punktionskanüle (51) nach Anspruch 1, dadurch gekennzeichnet, daß der Trokar oder die Punktionskanüle (51) mit dem patientenabgewandten Ende in einem axial verfahrbaren Schlitten (53) der Vorrichtung (50) ortsfest haltbar gelagert ist, daß der Schlitten (53) in beliebig langen vorgebbaren Wegabschnitten innerhalb der Vorrichtung (50) verschiebbar ist, daß der Trokar oder die Punktionskanüle (51) mit dem patientenseitigen Ende beim Verfahren des Schlittens (53) in der ersten Richtung (52) aus der Vorrichtung (50) heraustritt, und daß an der Vorrichtung (50) einenends die Anlagefläche (55) ausgebildet ist, aus der der Trokar oder die Punktionskanüle (51) beim Verfahren in der ersten Richtung 852) heraustritt und anderenends ein ortsfester und ein beweglicher Haltegriff (58, 59) ausgebildet ist, wobei über den beweglichen Haltegriff (59) die Verschiebung des Schlittens (53) in die erste Richtung (52) auslösbar ist.

9. Vorrichtung zur kontrollierten Plazierung eines Trokars oder einer Punktionskanüle (51) nach Anspruch 8, dadurch gekennzeichnet, daß der Schlitten (53) in einem halbschalenförmigen Gehäuse (56) der Vorrichtung (50) geführt gelagert ist und eine Einspannvorrichtung für das patientenabgewandte Ende des Trokars oder der Punktionskanüle (51) aufweist.

10. Vorrichtung zur kontrollierten Plazierung eines Trokars oder einer Punktionskanüle (51) nach Anspruch 9, dadurch gekennzeichnet, daß das halbschalenförmige Gehäuse (56) am patientenabgewandten Ende um die Längsachse der Vorrichtung (50) in Pfeilrichtungen (57) drehbar gelagert ist.

11. Vorrichtung zur kontrollierten Plazierung eines Trokars oder einer Punktionskanüle (51) nach Anspruch 8, dadurch gekennzeichnet, daß über den Auslenkweg des beweglichen Haltegriffs (59) zum ortsfesten Haltegriff (58) eine Führungsstange (60) verschiebbar ist, die mit dem Schlitten (53) eine feste Verbindung aufweist.

12. Vorrichtung zur kontrollierten Plazierung eines Trokars oder einer Punktionskanüle (51) nach Anspruch 11, dadurch gekennzeichnet, daß die Führungsstange (60) in jeder Positionslage unverrückbar gehalten ist und ausschließlich über die Auslösemittel in die (58, 59) erste Richtung (52) bewegbar ist.

13. Vorrichtung zur kontrollierten Plazierung eines Trokars oder einer Punktionskanüle (51) nach einem der Ansprüche 8 bis 12, dadurch gekennzeichnet, daß die Anlagefläche (55) einen Führungsabschnitt, beispielsweise als Ausnehmung (61) ausgebildet, aufweist, in dem der Trokar oder die Punktionskanüle (51) geführt verschiebbar ist.

14. Vorrichtung zur kontrollierten Plazierung eines Trokars oder einer Punktionskanüle (51) nach einem der Ansprüche 8 bis 13, dadurch gekennzeichnet, daß die Haltegriffe (58, 59) einen Entriegelungsmechanismus, beispielsweise einen Hebel (62), aufweisen, der im aktivierten Zustand eine Verschiebung des Schlittens (53) in eine zweite Richtung (63) ermöglicht.

15. Vorrichtung zur kontrollierten Plazierung eines Trokars oder einer Punktionskanüle (51) nach einem der Ansprüche 8 bis 14, dadurch gekennzeichnet, daß der Schlitten (53) Ausnehmungen (65) aufweist, in die das patientenabgewandte Ende des Trokars oder der Punktionskanüle (51) weitgehend formschlüßig einlegbar ist, und daß der Trokar oder die Punktionskanüle (51) über einen Schnellverschluß in den Ausnehmungen (65) richtungsstabil geführt gehalten ist.

16. Vorrichtung zur kontrollierten Plazierung eines Trokars oder einer Punktionskanüle (51) nach einem der Ansprüche 8 bis 15, dadurch gekennzeichnet, daß der Schlitten (53) und/oder die Anlagefläche (55) in der Vorrichtung (50) austauschbar gehalten sind.

17. Vorrichtung zur kontrollierten Plazierung eines Trokars oder einer Punktionskanüle (51) nach einem der Ansprüche 8 bis 16, dadurch gekennzeichnet, daß zwischen dem Schlitten (53) und den Auslösemitteln (58, 59) ein Abstandshalter (73) vorgesehen ist.

18. Vorrichtung zur kontrollierten Plazierung eines Trokars oder einer Punktionskanüle (11; 33; 51) nach einem der Ansprüche 1 bis 17, dadurch gekennzeichnet, daß die Vorrichtung (10; 30; 50) aus dampfsterilisierbaren Werkstoffen hergestellt ist, und daß die einzelnen Komponenten der Vorrichtung (10; 30; 50) einsehbar und zugänglich angeordnet sind.

## Claims

1. Device for the controlled positioning of a trocar or a hollow puncturing needle (11; 33; 51), wherein the trocar or hollow puncturing needle (11; 33; 51) is held in the device (10; 30; 50) in an axially displaceable fashion and is stepwise movable in the first direction (16; 43; 52) relative to a support surface (20; 44; 55) of the device (10; 30; 50) via activation means having a fixed handle (13; 31; 58) and a movable handle (14; 32; 59), wherein the displacement path of the movable handle (14; 32; 59) relative to the fixed handle (13; 31; 58) defines the length of the travel path of the trocar or of the hollow puncturing needle (11; 33; 51) in a first direction (16; 43; 52) towards the patient.

2. Device for the controlled positioning of a trocar or of a hollow puncturing needle (11) according to claim 1, characterized in that the trocar or hollow puncturing needle (11) to be positioned can be inserted into an aperture (21) of the device (10) and can be pushed through the device (10) and the trocar or the hollow puncturing needle (11) can be chocked with sections of an opening (22) of a plate (23) which is deflectable via the movable handle (14) by displacement of the movable handle (14) in a direction towards the fixed handle (13) and the trocar or the hollow puncturing needle (11) can be moved in the first direction (16) through displacement of the plate (23).

3. Device for the controlled positioning of a trocar or of a hollow puncturing needle (11) according to one of the claims 1 or 2, characterized in that the plate (23) is spring-loaded in a second direction (24) opposite to the first direction (16) and the maximum displacement of the plate (23) is limited by the displacement path between the movable handle (14) and the fixed handle (13).

4. Device for the controlled positioning of a trocar or of a hollow puncturing needle (11) according to one of the claims 2 or 3, characterized in that the device (10) can be swung-open at its upper side for providing access to the aperture (21).

5. Device for the controlled positioning of a trocar or of a hollow puncturing needle (11; 33) according to one of the claims 2 through 4, characterized in that the device (10; 30) has a mounting (19; 45) formed on a housing (12; 30') of the device (10; 30) opposite the handles (13, 14; 31, 32).

6. Device for the controlled positioning of a trocar or of a hollow puncturing needle (33) according to claim 1, characterized in that the trocar or the hollow puncturing needle (33) has recesses (36) on a limited outer peripheral section along the axial extent which engage in the device (30) in snap elements (37, 38), wherein the first snap element (37) is attached to the movable handle (32) and can be pivoted with the movable handle (32) and the second snap means (38) is disposed on the fixed housing (30') of the device (30).

7. Device for the controlled positioning of a trocar or of a hollow puncturing needle according to one of the claims 1 through 6, characterized in that the activation means (13, 14; 31, 32) are held apart by means of a spring (28; 34) and the activation means (13, 14; 31, 32) can be pushed towards each other in opposition to the force of the spring (28; 34).

8. Device for the controlled positioning of a trocar or of a hollow puncturing needle (51) according to claim 1, characterized in that the trocar or the hollow puncturing needle (51) is borne in a stationary fashion at its end facing away from the patient within an axially movable carriage (53) of the device (50) and the carriage (53) can be displaced along predetermined paths of arbitrary length within the device (50) and the trocar or the hollow puncturing needle (51) leaves the device (50) in the first direction (52) at the side facing the patient when the carriage (53) is moved, and the support surface (55), through which the trocar or the hollow puncturing needle (51) protrudes during movement in the first direction (52), is formed on the device (50) at one end and a stationary and a movable handle (58, 59) are disposed at the other end, wherein the displacement of the carriage (53) in the first direction (52) can be activated via the movable handle (59).

9. Device for the controlled positioning of a trocar or of a hollow puncturing needle (51) according to claim 8, characterized in that the carriage (53) is borne in a guided fashion within a half-shell-shaped housing (56) of the device (50) and has a tensioning device for the end of the trocar or of the hollow puncturing needle (51) facing away from the patient.

10. Device for the controlled positioning of a trocar or of a hollow puncturing needle (51) according to claim 9, characterized in that the half-shell-shaped housing (56) can be rotated about the longitudinal axis of the device (50) in the direction of arrow (57) at the end facing away from the patient.

11. Device for the controlled positioning of a trocar or of a hollow puncturing needle (51) according to claim 8, characterized in that a guide rod (60) is displaceable via the displacement path of the movable handle (59) towards the fixed handle (58) and has a fixed connection to the carriage (53).

12. Device for the controlled positioning of a trocar or of a hollow puncturing needle (51) according to claim 11, characterized in that the guide rod (60) is held without slippage at each positional location and can be moved exclusively via the activation means (58, 59) in the first direction (52).

13. Device for the controlled positioning of a trocar or of a hollow puncturing needle (51) according to claims 8 through 12, characterized in that the support surface (55) has a guiding section formed, for example, as a recess (61) in which the trocar or the hollow puncturing needle (51) is displaceable in a guided fashion.

14. Device for the controlled positioning of a trocar or of a hollow puncturing needle (51) according to one of the claims 8 through 13, characterized in that the handles (58, 59) have an unlocking mechanism, for example a lever (62), which facilitates, in the activated state, a displacement of the carriage (53) in a second direction (63).

15. Device for the controlled positioning of a trocar or of a hollow puncturing needle (51) according to one of the claims 8 through 14, characterized in that the carriage (53) has recesses (65) into which the end of the trocar or of the hollow puncturing needle (51) facing away from the patient can be inserted in a largely interlocking fashion and the trocar or the hollow puncturing needle (51) can be held and guided in a stable direction in the recesses (65) via a quick acting closure.

16. Device for the controlled positioning of a trocar or of a hollow puncturing needle (51) according to one of the claims 8 through 15, characterized in that the carriage (53) and/or the support surface (55) is held in the device (50) in an exchangeable fashion.

17. Device for the controlled positioning of a trocar or of a hollow puncturing needle (51) according to one of the claims 8 through 16, characterized in that a spacer (73) is provided for between the carriage (53) and the activation means (58, 59).

18. Device for the controlled positioning of a trocar or of a hollow puncturing needle (11; 33; 51) according to one of the claims 1 through 17, characterized in that the device (10; 30; 50) is produced from steam-sterilizable materials and the individual components of the device (10; 30; 50) are arranged in a visible and accessible fashion.

## Revendications

1. Dispositif pour la mise en place contrôlée d'un trocart ou d'une canule de ponction (11 ; 33 ; 51), dans lequel le trocart ou la canule de ponction (11 ; 33 ; 51) est tenu de façon à pouvoir se déplacer axialement dans le dispositif (10 ; 30 ; 50) et peut être déplacé pas à pas par rapport à une surface d'appui (20 ; 44 ; 55) du dispositif et dans une première direction (16 ; 43 ; 52) dirigée vers le patient, au moyen d'organes de déclenchement, qui comprennent une poignée de maintien fixe (13 ; 31 ; 58) et une poignée de maintien mobile (14 ; 32 ; 59), et la longueur de la course de déplacement du trocart ou de la canule de ponction (11 ; 33 ; 51) est définie dans la première direction au moyen du trajet de déplacement de la poignée de maintien mobile (14 ; 32 ; 59) par rapport à la poignée de maintien fixe (13 ; 31; 58).

2. Dispositif pour la mise en place contrôlée d'un trocart ou d'une canule de ponction (11), selon la revendication 1, caractérisé en ce que le trocart ou la canule de ponction (11) à placer est susceptible d'être introduit dans une ouverture (21) du dispositif (10) et d'être déplacé à travers le dispositif (10), en ce que le trocart ou la canule de ponction (11) est susceptible d'être coincé, par déflexion de la poignée de maintien mobile (14) en direction de la poignée de maintien fixe (13), avec des tronçons d'une traversée (22) d'une plaque (23) susceptible d'être déviée au moyen de la poignée de maintien mobile (14), et en ce que le trocart ou la canule de ponction (11) peut être déplacé dans la première direction (16) par déviation de la plaque (23).

3. Dispositif pour la mise en place contrôlée d'un trocart ou d'une canule de ponction (11), selon l'une ou l'autre des revendications 1 et 2, caractérisé en ce que la plaque (23) est sollicitée par un ressort dans une seconde direction (24) opposée à la première direction (16), et la déviation maximum de la plaque (23) est limitée par la déviation de la poignée de maintien mobile (14) par rapport à la poignée de maintien fixe (13).

4. Dispositif pour la mise en place contrôlée d'un trocart ou d'une canule de ponction (11) selon l'une ou l'autre des revendications 2 et 3, caractérisé en ce que le dispositif (10) est susceptible d'être basculé vers le haut sur la face supérieure pour libérer l'ouverture (21).

5. Dispositif pour la mise en place contrôlée d'un trocart ou d'une canule de ponction (11 ; 33) selon l'une des revendications 2 à 4, caractérisé en ce que le dispositif (10 ; 30) comporte à l'opposé des poignées de maintien (13 ; 14 ; 31 ; 32) une monture (19 ; 45) réalisée sur le boîtier (12 ; 30') du dispositif (10 ; 30).

6. Dispositif pour la mise en place contrôlée d'un trocart ou d'une canule de ponction (33) selon la revendication 1, caractérisé en ce que le trocart ou la canule de ponction (33) comporte sur un tronçon périphérique extérieur limité le long de son extension axiale des entailles (36) qui attaquent des éléments d'enclenchement (37, 38) dans le dispositif (30), le premier élément d'enclenchement (37) étant fixé sur la poignée de maintien mobile (32) et capable de basculer avec la poignée de maintien mobile (32), et le second élément d'enclenchement (38) étant agencé sur le boîtier fixe (30') du dispositif (30).

7. Dispositif pour la mise en place contrôlée d'un trocart ou d'une canule de ponction, selon l'une des revendications 1 à 6, caractérisé en ce que les moyens de déclenchement (13, 14 ; 31, 32) sont tenus en écartement au moyen de ressorts (28 ; 34), et en ce que les moyens de déclenchement (13, 14 ; 31, 32) sont mobiles en direction l'un de l'autre à l'encontre de la force des ressorts (28 ; 34).

8. Dispositif pour la mise en place contrôlée d'un trocart ou d'une canule de ponction (51) selon la revendication 1, caractérisé en ce que le trocart ou la canule de ponction (51) est monté de façon à pouvoir être tenu à un emplacement fixe, avec l'extrémité détournée du patient, dans un chariot (53) axialement mobile du dispositif (50), en ce que le chariot (53) est susceptible d'être déplacé à l'intérieur du dispositif (50) sur des tronçons de trajet prédéterminés de longueurs quelconques, en ce que le trocart ou la canule de ponction (51) sort par l'extrémité côté patient hors de l'appareil du dispositif (50) lorsqu'on déplace le chariot (53) dans la première direction (52), et en ce que sur le dispositif (50) à une extrémité est réalisée la surface d'appui (55) hors de laquelle sort le trocart ou la canule de ponction (51) lors du déplacement dans la première direction (52), et à l'autre extrémité sont réalisées une poignée de maintien fixe (58) et une poignée de maintien mobile (59), et dans lequel le déplacement du chariot (53) dans la première direction (52) peut être déclenché au moyen de la poignée de maintien mobile (59).

9. Dispositif pour la mise en place contrôlée d'un trocart ou d'une canule de ponction (51), selon la revendication 8, caractérisé en ce que le chariot (53) est monté de façon guidée dans un boîtier (56), en forme de demi-coque, du dispositif (50) et comporte un dispositif de serrage pour l'extrémité détournée du patient du trocart ou de la canule de ponction (51).

10. Dispositif pour la mise en place contrôlée d'un trocart ou d'une canule de ponction (51), selon la revendication 9, caractérisé en ce que le boîtier en forme de demi-coque (56) est monté, à l'extrémité détournée du patient, en rotation autour de l'axe longitudinal du dispositif (50) dans les directions des flèches (57).

11. Dispositif pour la mise en place contrôlée d'un trocart ou d'une canule de ponction (51), selon la revendication 8, caractérisé en ce qu'une tige de guidage (60), qui présente une liaison fixe avec le chariot (53), est déplaçable sur le trajet de déflexion de la poignée de maintien mobile (59) par rapport à la poignée de maintien fixe (58).

12. Dispositif pour la mise en place contrôlée d'un trocart ou d'une canule de ponction (51), selon la revendication 11, caractérisé en ce que la tige de guidage (60) est tenue dans chaque position sans possibilité de se déplacer, et peut être déplacée dans la première direction (52) exclusivement à l'aide des moyens de déclenchement.

13. Dispositif pour la mise en place contrôlée d'un trocart ou d'une canule de ponction (51), selon l'une des revendications 8 à 12, caractérisé en ce que la surface d'appui (55) comporte un tronçon de guidage, réalisé par exemple sous forme d'un évidement (61), dans lequel le trocart ou la canule de ponction (51) peut être déplacé de façon guidée.

14. Dispositif pour la mise en place contrôlée d'un trocart ou d'une canule de ponction (51), selon l'une des revendications 8 à 13, caractérisé en ce que les poignées de maintien (58, 59) comprennent un mécanisme de déverrouillage, par exemple un levier (62) qui permet dans l'état activé un déplacement du chariot (52) dans une seconde direction (63).

15. Dispositif pour la mise en place contrôlée d'un trocart ou d'une canule de ponction (51), selon l'une des revendications 8 à 14, caractérisé en ce que le chariot (53) comprend des évidements (65), dans lesquels l'extrémité détournée du patient du trocart ou de la canule de ponction (51) est susceptible d'être mise en place largement en coopération de formes, et en ce que le trocart ou la canule de ponction (51) est tenu et stablement guidé en direction dans les évidements (65) à l'aide d'une fermeture rapide.

16. Dispositif pour la mise en place contrôlée d'un trocart ou d'une canule de ponction (51), selon l'une des revendications 8 à 15, caractérisé en ce que le chariot (53) et/ou la surface d'appui (55) sont tenus de façon interchangeable dans le dispositif(50).

17. Dispositif pour la mise en place contrôlée d'un trocart ou d'une canule de ponction (51), selon l'une des revendications 8 à 16, caractérisé en ce qu'un élément d'écartement (73) est prévu entre le chariot (53) et les moyens de déclenchement (58, 59).

18. Dispositif pour la mise en place contrôlée d'un trocart ou d'une canule de ponction (11 ; 33 ; 51), selon l'une des revendications 1 à 17, caractérisé en ce que le dispositif (10 ; 30 ; 50) est réalisé en matériaux susceptibles d'être stérilisés à la vapeur, et en ce que les composants individuels du dispositif (10 ; 30 ; 50) sont agencés de façon à pouvoir être inspectés et accessibles.
